# EUROPEAN PATENT APPLICATION

(11) **EP 4 478 047 A2**
(43) Date of publication of application: **18.12.2024**
(21) Application number: 24180012.7
(22) Date of filing: 04.06.2024
(51) Int. Cl.: G01N 33/00

(54) **CROSS CALIBRATION BY AUTOMATIC GAS DETECTORS**

(30) Priority: 12.06.2023 GB 202308760
(71) Applicant: Crowcon Detection Instruments Limited, Abingdon, Oxfordshire OX14 4SD (GB)
(72) Inventor: BASHAM, Paul, High Wycombe, HP13 6BT (GB)
(74) Representative: Black, Diego

(57) **Abstract**

A method of automatically calibrating a toxic gas detector, wherein the gas detector comprises one or more electrochemical toxic gas sensors, each electrochemical toxic gas sensor configured to detect the presence of a different target toxic gas, said toxic gas detector in communication with a processor and a memory, wherein the method comprises: determining a calibration factor for each of the one or more electrochemical toxic gas sensors in the toxic gas detector, wherein the determining of the calibration factor for each of the plurality of electrochemical toxic gas sensors comprises the steps of: providing a predetermined concentration of the target toxic gas to the electrochemical toxic gas sensor and measuring a response of the electrochemical toxic gas sensor to the target toxic gas, wherein the target toxic gas is different for each of the one or more electrochemical toxic gas sensors; providing a predetermined concentration of an electrochemical calibration gas to the electrochemical toxic gas sensor and measuring a response of the electrochemical toxic gas sensor to the electrochemical calibration gas, wherein the electrochemical calibration gas is the same for each of the one or more electrochemical toxic gas sensors; calculating the calibration factor as a function of the response of the electrochemical toxic gas sensor to the target toxic gas and the electrochemical calibration gas; storing the calibration factor in the memory; and calibrating the toxic gas detector by: supplying a predetermined concentration of the electrochemical calibration gas to each of the one or more electrochemical toxic gas sensors and measuring the response of each of the one or more electrochemical toxic gas sensors to the electrochemical calibration gas; and calibrating the response of each of the one or more electrochemical toxic gas sensors to the corresponding target gas based on the respective stored calibration factor for the electrochemical toxic gas sensor and the measured response to the electrochemical calibration gas.

## Description

### TECHNICAL FIELD

The present disclosure relates to a device and method for calibrating a gas detector. Particularly, but not exclusively, it relates to calibrating a gas detector comprising multiple different sensors using a single calibration gas.

### BACKGROUND

Gas detectors are operated to measure the presence of potentially dangerous gases to ensure a user is not exposed to a dangerous level of gas. When exposed to high concentrations of toxic or flammable gas, or low concentrations of oxygen, the gas detector warns a user by means of sounders, alarm LEDs and vibrations. Typically, users of gas detectors will perform an automatic calibration process daily to ensure that the detector is correctly functioning. The calibration process typically involves providing an amount of gas of the type or types to be detected to test the response of the sensors within the portable gas detector. This requires testing each sensor with a corresponding gas which the sensor is configured to detect.

However, some gases may deaden some of the other types of sensors within the device and further the user needs to carry multiple gas cylinders to test each different sensor with the appropriate gas. The cost of calibrating detectors can be expensive as it can require significant amounts of gas to be used to calibrate gas detectors which detect multiple types of gases.

Accordingly, the ability to improve the calibration process of a gas detector is desirable.

An object of the present invention is to mitigate some of the deficiencies of the prior art mentioned above.

### SUMMARY OF THE INVENTION

In order to address some of the issues with the prior art there is provided a method of automatically calibrating a toxic gas detector, wherein the gas detector comprises a plurality of electrochemical toxic gas sensors, each electrochemical toxic gas sensor configured to detect the presence of a different target toxic gas, said toxic gas detector in communication with a processor and a memory, wherein the method comprises:
determining a calibration factor for each of the plurality of electrochemical toxic gas sensors in the toxic gas detector, wherein the determining of the calibration factor for each of the plurality of electrochemical toxic gas sensors comprises: providing an amount of the target toxic gas to the electrochemical toxic gas sensor and measuring a response of the electrochemical toxic gas sensor to the target toxic gas, wherein the target toxic gas is different for each of the plurality of electrochemical toxic gas sensors;
providing an amount of an electrochemical calibration gas to the electrochemical toxic gas sensor and measuring a response of the electrochemical toxic gas sensor to the electrochemical calibration gas, wherein the electrochemical calibration gas is the same for each of the plurality of electrochemical toxic gas sensors; calculating the calibration factor as a function of the response of the electrochemical toxic gas sensor to the target toxic gas and the electrochemical calibration gas; storing the calibration factor in the memory; and calibrating the toxic gas detector by: supplying an amount of the electrochemical calibration gas to each of the plurality electrochemical toxic gas sensors and measuring the response of each of the plurality of electrochemical toxic gas sensors to the electrochemical calibration gas; and calibrating the response of each of the plurality electrochemical toxic gas sensors to the corresponding target gas based on the respective stored calibration factor for the electrochemical toxic gas sensor and the measured response to the electrochemical calibration gas.

Such a method allows for the use of a single calibration gas to be used to calibrate multiple toxic gas sensors in a device. Therefore, the calibration process is quicker, and less expensive, as single calibration gas can be used. Furthermore, as the calibration factor is determined for each sensor individually the manufacturing process of the detector becomes cheaper. This is explained in further detail below.

Other aspects of the invention will be apparent from the appended claim set.

### BRIEF DESCRIPTION OF THE DRAWINGS

One or more embodiments of the invention will now be described, by way of example only, with reference to the accompanying drawings, in which:
Figure 1 is a schematic representation of the apparatus according to an aspect of the invention;
Figure 2 is a flow chart of the process of calibrating a gas detector according to an aspect of the invention;
Figure 3 is a plot of the accuracy of a number of commercially available ammonia sensors to a known concentration of ammonia; and
Figure 4 is an example of a bump testing unit used in conjunction with the present invention.

### DETAILED DESCRIPTION

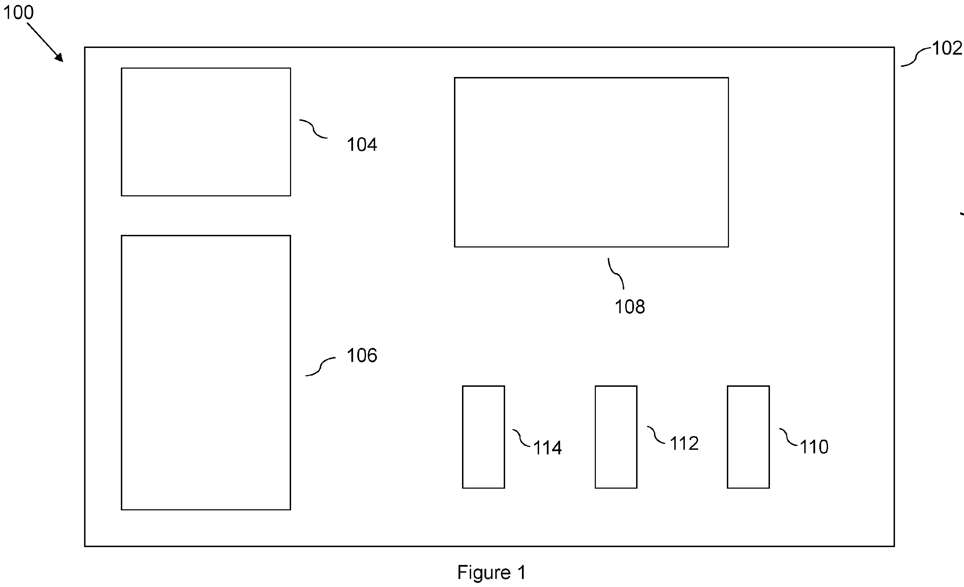

The present invention provides a device and method capable of calibrating multiple gas sensors in a toxic gas detector by a single exposure to a calibration gas. Such a gas detector is shown in Figure 1 in accordance with the claimed invention.

Figure 1 shows a schematic of a toxic gas detector 100. The toxic gas detector 100 comprises at least a housing 102 in which there is a plurality of toxic gas sensors 110, 112, 114. The toxic gas sensors are electrochemical toxic gas sensors, and accordingly references to toxic gas sensors are to be understood as electrochemical toxic gas sensors. That is to say the toxic gas sensor is an electrochemical sensor configured to detect toxic gas.

The toxic gas detector 100 is shown in Figure 1 as comprising three toxic gas sensors 110, 112, 114 as an example only. Any number of gas sensors can be comprised in the gas detector as suitable for its intended use. For example, the toxic gas detector 100 may comprise two different gas sensors.

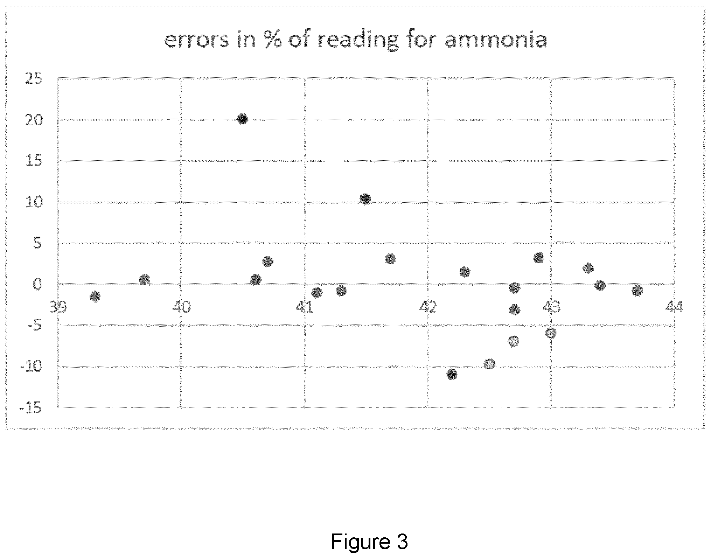

The gas detector is a toxic gas detector. Toxic gases are gases which are gases which produce hazardous physiological effects when inhaled. Known examples of toxic gases include ammonia, sulphur dioxide, chlorine, hydrogen fluoride, nitrogen monoxide, hydrogen chloride, ozone, chlorine dioxide, ethylene oxide, phosgene, hydrogen cyanide, arsine, phosphine, silane. The present disclosure is specifically directed to toxic gas detectors as toxic gas detectors, as explained with reference to Figure 3 and associated description below, show significant variability which can make their calibration difficult.

The toxic gas sensors 110, 112, 114 are electrochemical gas sensors. For example, the gas sensors may include one or more of an ammonia, sulphur dioxide, chlorine, hydrogen fluoride, nitrogen monoxide, hydrogen chloride, ozone, chlorine dioxide, ethylene oxide, phosgene, hydrogen cyanide, arsine, phosphine, and/or silane gas sensor. Any combination of these gas sensors can be used in a single toxic gas detector 100.

The electrochemical toxic gas sensors 110, 112, 114 used in the toxic gas detector 100 are known, commercially available electrochemical gas sensors and their form and functionality is known in the art.

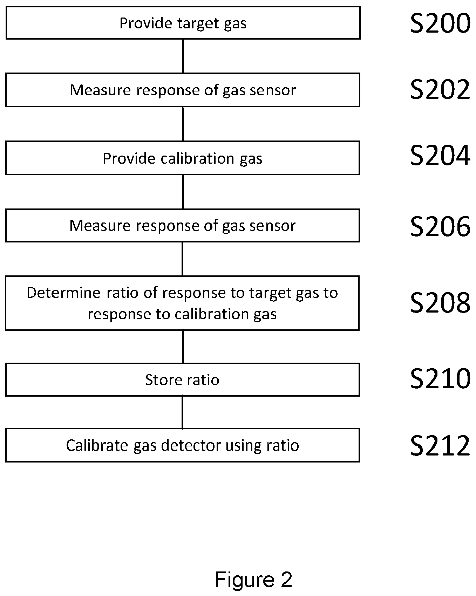

Each of the above-mentioned electrochemical gas sensors have a cross sensitivity with other electrochemical gasses. Therefore, when an electrochemical gas sensor, say an ammonia sensor, is exposed to a different electrochemical gas, say nitrogen dioxide, it will react and produce a reading to the different electrochemical gas. As the electrochemical gas sensor is not optimised to detect the different electrochemical gas the level of reading, or detection, is lower than for the target gas to which the electrochemical gas detector is calibrated. If the response is known, then such a gas can be used to calibrate the device, how as explained below given issues with toxic gas detectors there are challenges in such calibration, which the present invention seeks to address. Preferably gases such as nitrogen dioxide or hydrogen sulphide are used as these are non-explosive gases. As the sensors are all sensitive to such electrochemical gases, one of the electrochemical gases can be used for all of the gas sensors in the toxic gas detector 100. The calibration process is discussed in detail in relation to Figure 2.

However, it is found that different types of electrochemical gas detectors have different cross sensitivities to target gases. Furthermore, it is also found that batches of the same commercially available detector may show variability in the sensitivity to the target gas and to the cross sensitivity to the same calibration gas. This is shown in Figure 3.

Figure 3 is a plot of preinstallation testing for a number of commercially available ammonia sensors. Each dot represents an individual ammonia sensor, with the value determined by the sensor along the x- axis and the relative error of the reading on the y-axis.

As can be seen each ammonia sensor, despite being of the same make and model, has a different response. As can be seen a significant number of sensors show relative error readings of ±5%, with the relative error of ±5% being an industry standard of an acceptable error reading. Typically, sensors which show a relative error beyond the ±5% range are rejected leading to increased production costs. Furthermore, variability in cross sensitivities to other electrochemical target gases is seen in such samples and the relative error is typically much higher ±20%. Additionally, it is also known that such detectors will deteriorate in their response to the target gas, and other cross sensitive gases, over time. Therefore, such variability can cause issues with using cross sensitivities for toxic gas detection. The process for overcoming such issues is described with reference to Figure 2.

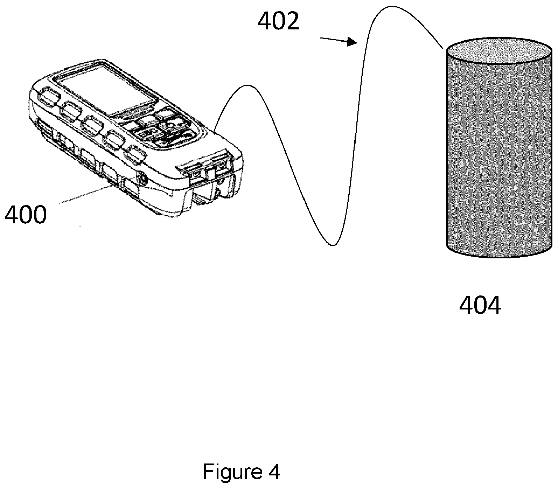

Returning to Figure 1, preferably, the toxic gas detector 100 comprises an ammonia gas sensor, a sulphur dioxide gas sensor, and a chlorine gas sensor in a single device. Other configurations of the toxic gas detector 100, in terms of the types of sensor can be used. Such a device is preferably calibrated with nitrogen dioxide, or hydrogen sulphide as the electrochemical calibration gas. Calibration gases such as nitrogen dioxide, or hydrogen sulphide, are preferred as they are relatively stable and do not deteriorate as quickly as other electrochemical gases. Such deterioration of the gas can introduce further errors into the calibration process. When the toxic gas detector 100 is used in a gassing station, such as one shown in Figure 4, the use of a chemically stable electrochemical gas is preferred as the gas typically has to travel through tubing which can accelerate the deterioration of the gas.

The toxic gas detector 100 further comprises a processor 104 and a memory 106. The memory 106 is preferably non-volatile memory and the processor 104 is configured to write date, such as determined calibration data to the memory 106. The memory 106 is capable of storing data when the toxic gas detector 100 is deactivated.

The toxic gas detector 100 can also have a display 108 allowing calibration or exposure data to be communicated to a user. The display 108 may be an LCD screen, for example, or any other known suitable means of display. The display 108 is preferably active during calibration of the device.

The functionality of the toxic gas detector 100 in particular in the calibration phase is described below with reference to Figure 2.

Figure 2 is a flow chart of the process of calibrating a gas detector, such as toxic gas detector 100, according to an aspect of the invention.

As described above, the variability of individual electrochemical gas detectors even from the same manufacturer to the same target gas makes their calibration more difficult. Additionally, the variability in cross sensitivity to different electrochemical gases for toxic gas sensors tends to be high, with errors of ±20% being common. The process described in Figure 2 allows for the calibration of electrochemical toxic gas sensors and furthermore addresses the issues with variability of different sensors.

In a first step S200, an amount of the target toxic gas is provided to a toxic gas sensor to begin the calibration process for the toxic gas sensor. This step preferably occurs during a testing stage before incorporation into the toxic gas detector 100. As each toxic gas sensor is individually calibrated the target toxic gas is provided to each individual sensor. As will be apparent below, by calibrating each individual toxic gas sensor in the process of Figure 2, the issues associated with the variability of toxic gas detectors are addressed.

The target gas is chosen dependent on the properties of the toxic gas sensor 110, 112, 114. If the toxic gas sensor 110 is an ammonia gas sensor, then the chosen target gas is ammonia. Similarly, if the toxic gas sensor 110 is a sulphur dioxide gas sensor, then the chosen target gas is sulphur dioxide. This step applies to each of the gas sensors discussed with reference to Figure 1.

Preferably the concentration of the target gas provided is dependent on the target gas being detected and the toxic gas sensor 110. Ammonia sensors are configured to detect concentrations in the range up to 1000ppm, preferably in the range 0-100ppm. Sulphur dioxide sensors are configured to detect concentrations in the range up to 500ppm, preferably in the range 0-10ppm. Chlorine sensors are configured to detect concentrations in the range up to 50ppm, preferably in the range 0-10ppm. Hydrogen fluoride sensors are configured to detect concentrations in the range up to 10ppm, preferably in the range 0-10ppm. Nitrogen monoxide sensors are configured to detect concentrations in the range up to 500ppm, preferably in the range 0-50ppm. Hydrogen chloride sensors are configured to detect concentrations in the range up to 30ppm, preferably in the range 0-10ppm. Ozone sensors are configured to detect concentrations in the range up to 10ppm, preferably in the range 0-1ppm. Chlorine dioxide sensors are configured to detect concentrations in the range up to 10ppm, preferably in the range 0-1ppm.

For each toxic gas sensor 110, 112, 114, the corresponding target gas is provided to that sensor and at step S202, the response of the gas sensor to that target gas is measured and recorded. As shown in Figure 3 as the response of a detector may vary, this process is repeated for each detector. The process of measuring and recording the response of the toxic gas sensor to the target gas is known and occurs in the known manner.

Each of the toxic gas sensors 110, 112, 114 tested at step S202 are then exposed to an electrochemical calibration gas at step S204. For example, the electrochemical calibration gas can be nitrogen dioxide or hydrogen sulphide. The toxic gas sensors 110, 112, 114 are typically exposed to a concentration of calibration gas of less than 25ppm.

Each of the toxic gas sensors 110, 112, 114 which are to be included in a toxic gas detector 100 are preferably exposed to the same calibration gas. When the sensors are installed in the toxic gas detector 100 this reduces the number of calibration gases, and thus the number of gas cylinders a user of the device must provide when calibrating the toxic gas detector 100.

At step S206, the response of each toxic gas sensor 110, 112, 114 to the calibration gas is measured and recorded.

As the response for each detector may vary the process occurs for each sensor thus ensuring that any variability in the sensor is determined and accounted for.

A ratio of the target gas measurement to the calibration gas measurement is then determined for each toxic gas sensor 110, 112, 114 at step S208. This ratio is stored in memory 106 of toxic gas detector 100 at step S210.

Optionally, steps S204, S206, S208 and S210 are repeated for multiple electrochemical calibration gases. This allows for multiple electrochemical calibration gases to be supplied either sequentially, or simultaneously, and for multiple toxic gas sensors to be calibrated either sequentially, or simultaneously.

The ratio of the response between the target gas and the electrochemical calibration gas is found to remain consistent for each detector. Whilst individual detectors may show variations in response when compared to other detectors, the inventors have beneficially found that the response between the target gas and the calibration gas for each detector will remain constant. Therefore, the ratio of the response to the target gas and the calibration gas can be used in future measurements to calibrate the detector.

As such the ratio, or a value which is indicative of the ratio, along with an identifier of the toxic gas detector is stored in the memory of the toxic gas detector 100. Preferably, the identifier associated with the toxic gas detector is unique or a quasi-identifier allowing the ratio to be readily associated with the detector for which it has been calibrated. Preferably, an indication of the electrochemical calibration gas is also stored in the memory of the toxic gas detector 100 thereby allowing multiple electrochemical calibration gases to be used.

A further advantage is that as the toxic gas detector has been calibrated with a known concentration of the target gas at step S202, the relative error between the amount of detected gas at step S204 and the amount supplied can also be used to provide a correction factor in the calibration process. This correction factor is used to scale the ratio and stored in the memory. For example, when it is known from previous measurements that there is a known error for a particular sensor for a given gas subsequent measures of the same toxic gas can be corrected by extracting the known error for the memory and using the error in the calculation in a known manner.

At step S212 the toxic gas detector 100 is calibrated, for example during a daily bump test, or during normal use. At step S212 the toxic gas detector 100 is exposed to the same electrochemical gas used at step S204, or if the gas detector has been calibrated with multiple electrochemical gases an indication of the electrochemical gas is provided to the toxic gas detector 100, for example via a user interface. Preferably, the toxic gas detector and its ratio are identified by the unique identifier, or quasi identifier.

The stored ratio as calculated at step S210 for the relevant electrochemical target gas is retrieved from the memory and used to calibrate the toxic gas detector 100 at step S212. As each toxic gas sensor 110, 112, 114 has a stored ratio related to their individual target gas compared to a common calibration gas, the calibration gas can be used to calibrate all of the toxic gas sensors 110, 112, 114 in the toxic gas detector 100. The ratio is used to scale a measured response to the calibration gas such that the toxic gas detector 100 can determine an equivalent concentration of target gas. For example, if at step S208 if it is determined that the ratio between an electrochemical gas and the calibration gas is 0.25 for a particular sensor, during the calibration stage at step S212 the response of the toxic detector to the electrochemical calibration gas is scaled accordingly (by a factor of 4) to determine the expected response of the detector to the toxic gas.

In further embodiments, the toxic gas detector 100 comprises communication means (not shown) such as Wi-Fi, or cellular communication means, to enable the toxic gas detector 100 to communicate with an external memory such as a database (not shown). The memory comprises the unique identifier, or quasi-identifier of the toxic gas sensors, and the determined ratios stored. Therefore, by using the communication means the toxic gas detector 100 is able to access the determined values.

As the concentration of the electrochemical calibration gas is known the error associated with the toxic gas detector can be determined, in that the expected response to the gas is known (as per step S202), and if there is a significant difference between the expected response and the determined response it would indicate that the toxic gas sensor may not be accurate and may require repair or recalibration.

Advantageously, the process is particularly effective for toxic gases. Toxic gas sensors show significant variability between individual sensors to both the target gas and any gas to which it has a cross sensitivity. This makes the use of cross sensitive gases in calibration, or bump testing, for toxic gas detectors unreliable. However, the inventors have beneficially realised that whilst the response between sensors may vary, each sensor maintains a consistent response in cross sensitivity between the target gas and a calibration gas. Therefore, by determining a ratio of response and storing that response for each sensor electrochemical calibration gases can be used.

A further advantage of the present system is that as multiple toxic gas sensors 110, 112, 114 in the toxic gas detector 100 are calibrated using the same electrochemical target gas the calibration, or bump testing process, during the calibration process, such as bump testing, it only requires the toxic gas detector 100 to be exposed to a single electrochemical calibration gas. That is to say there does not need to be a different calibration gas for each toxic gas sensor in the toxic gas detector. This reduces the amount of gas which needs to be used for each test, and furthermore speeds up the process of calibration and bump testing.

A further advantage is that when the toxic gas detector 100 is tested using a rig, such as one shown in Figure 4, the electrochemical calibration gas can be selected based on its properties. Figure 4 shows an example of a toxic gas detector 400 connected via tubing 402 to a cannister 404 of electrochemical calibration gas. The toxic gas detector 400 shown in Figure 4 is the same toxic gas detector 100 as described above. In use the cannister of electrochemical calibration gas 404 has a known concentration of a given electrochemical calibration gas. The cannister 404 is connected to the toxic gas detector 400 via tubing 402 in a known manner. The electrochemical calibration gas therefore passing through the tubing to the detector to perform the calibration, or bump test.

Preferably, electrochemical gases which do not show significant degradation when passing through the tubing 402, which is typically made of some form plastic, are selected. This allows for the selection of an electrochemical calibration gas which not only shows cross sensitivities in toxic gas detectors but also does not degrade in tubing. Such a selection ensures that the amount of gas needed to bump test one or more gas toxic detectors 100 is minimised. Preferably the electrochemical gas is either nitrogen dioxide or hydrogen sulphide as both gases satisfy both requirements. Preferably the electrochemical calibration gas is provided at a concentration of up to 25ppm as such a concentration allows for the toxic gas sensor to detect the gas whilst keeping the amount of gas needed to perform the test at a minimum.

## Claims

1. A method of automatically calibrating a toxic gas detector, wherein the toxic gas detector comprises a plurality of electrochemical toxic gas sensors, each electrochemical toxic gas sensor configured to detect a presence of a different target toxic gas, said toxic gas detector in communication with a processor and a memory, wherein the method comprises:
determining a calibration factor for each of the plurality of electrochemical toxic gas sensors in the toxic gas detector, wherein the determining of the calibration factor for each of the plurality of electrochemical toxic gas sensors comprises the steps of:
providing a predetermined concentration of the target toxic gas to the electrochemical toxic gas sensor and measuring a response of the electrochemical toxic gas sensor to the target toxic gas, wherein the target toxic gas is different for each of the plurality of electrochemical toxic gas sensors;
providing a predetermined concentration of an electrochemical calibration gas to the electrochemical toxic gas sensor and measuring a response of the electrochemical toxic gas sensor to the electrochemical calibration gas, wherein the electrochemical calibration gas is the same for each of the plurality of electrochemical toxic gas sensors;
calculating the calibration factor as a function of the response of the electrochemical toxic gas sensor to the target toxic gas and the electrochemical calibration gas;
storing the calibration factor in the memory; and
calibrating the toxic gas detector by:
supplying a predetermined concentration of the electrochemical calibration gas to each of the plurality of electrochemical toxic gas sensors and measuring the response of each of the one or more electrochemical toxic gas sensors to the electrochemical calibration gas; and
calibrating the response of each of the one or more electrochemical toxic gas sensors to the corresponding target gas based on the respective stored calibration factor for the electrochemical toxic gas sensor and the measured response to the electrochemical calibration gas.

2. The method of claim 1 wherein, wherein the target toxic gas for each of the plurality of electrochemical gas sensors is chosen from a group comprising: ammonia, sulphur dioxide, chlorine, hydrogen fluoride, nitrogen monoxide, hydrogen chloride, ozone, chlorine dioxide, ethylene oxide, phosgene, hydrogen cyanide, arsine, phosphine, silane.

3. The method of any preceding claim, wherein the electrochemical calibration gas is either nitrogen dioxide or hydrogen sulphide and/or wherein the electrochemical calibration gas is provided at a concentration up to 25ppm.

4. The method of any preceding claim, wherein one of the electrochemical toxic gas sensors is an ammonia sensor, preferably wherein the ammonia sensor is configured to detect concentrations of ammonia in the range of 0-100ppm.

5. The method of any preceding claim, wherein one of the electrochemical toxic gas sensors is a sulphur dioxide sensor, preferably wherein the sulphur dioxide sensor configured to detect concentrations of sulphur dioxide in the range of 0-10ppm.

6. The method of any preceding claim, wherein one of the electrochemical toxic gas sensors is a chlorine sensor, preferably wherein the chlorine sensor configured to detect concentrations of chlorine in the range of 0-10ppm.

7. The method of any preceding claim, wherein one of the electrochemical toxic gas sensors is a hydrogen fluoride sensor, preferably wherein the hydrogen fluoride sensor configured to detect concentrations of hydrogen fluoride in the range of 0-10ppm.

8. The method of any preceding claim, wherein one of the electrochemical toxic gas sensors is a nitrogen monoxide sensor, preferably wherein the nitrogen monoxide sensor configured to detect concentrations of nitrogen monoxide in the range of 0-50ppm.

9. The method of any preceding claim, wherein one of the electrochemical toxic gas sensors is a hydrogen chloride sensor, preferably wherein the hydrogen chloride sensor configured to detect concentrations of hydrogen chloride in the range of 0-10ppm.

10. The method of any preceding claim, wherein one of the electrochemical toxic gas sensors is an ozone sensor, preferably wherein the ozone sensor configured to detect concentrations of ozone in the range 0-1ppm.

11. The method of any preceding claim, wherein one of the electrochemical toxic gas sensors is a chlorine dioxide sensor, preferably wherein the chlorine dioxide sensor configured to detect concentrations of chlorine dioxide in the range 0-1ppm.

12. The method of any proceeding claim wherein a plurality of electrochemical calibration gases are supplied to the sensor in a predetermined concentration and the method further comprises calibrating the respective toxic gas sensor for each of the supplied plurality of electrochemical calibration gases.

13. A toxic gas detector in communication with a processor and a memory, the device comprising:
a plurality of electrochemical gas sensors, wherein each electrochemical gas sensor is configured to detect the presence of a different target gas and an electrochemical calibration gas, wherein the electrochemical calibration gas is the same for each electrochemical gas sensor,
the device configured to carry out the method steps of any preceding claim.

14. A toxic gas detector comprising:
a plurality of electrochemical gas sensors, each of the plurality of electrochemical toxic gas sensors configured to detect the presence of a different target toxic gas;
a memory, having stored thereon a calibration factor for each of the plurality of electrochemical toxic gas sensors, said calibration factor indicative of a predetermined response of the electrochemical toxic gas sensor to an electrochemical calibration gas, wherein the electrochemical calibration gas is the same for each of the one or more electrochemical toxic gas sensors; and
a processor configured to calibrate the plurality of electrochemical toxic gas sensors by:
determining a response for the plurality of electrochemical toxic gas sensors to a predetermined amount of the first electrochemical calibration gas; and
scaling the response for the plurality of electrochemical toxic gas sensors based on the determined response to the first electrochemical calibration gas and the calibration factor stored in the memory for the electrochemical toxic gas sensor.
